# EUROPEAN PATENT APPLICATION

(11) **EP 3 811 935 A1**
(43) Date of publication of application: **28.04.2021**
(21) Application number: 20214023.2
(22) Date of filing: 28.08.2015
(51) Int. Cl.: A61K 31/05, A61K 36/9066, A61K 9/08, A61K 9/06, A61K 9/70, A61P 3/04, A61Q 19/06, A61K 8/34, A61K 8/9794, A61K 8/35

(54) **COMPOSITION FOR REDUCING LOCAL FAT AND BODY WEIGHT, AND PHARMACEUTICALS AND USE THEREOF**

(30) Priority: 28.08.2014 US 201462042864 P
(62) Divisional of application: 15836003.2
(71) Applicant: Caliway Biopharmaceuticals Co. Ltd., New Taipei City 221 (TW)
(72) Inventor: LING, Yu-Fang, 221 New Taipei City (TW)
(74) Representative: Haseltine Lake Kempner LLP

(57) **Abstract**

Disclosed in the present invention is the composition for reducing local fat and body weight, and pharmaceuticals and use thereof. The composition contains resveratrol and curcumin extract at a weight ration from 1:30 to 10:1. The composition and pharmaceuticals thereof of the invention can inhibit the growth of fat cells, cause planned apoptosis of fat cells, achieve the effects of reducing fat cells, and reducing local fat deposition and body weight without causing inflammations or necrosis of surrounding cells or tissues and inflammations or pain reactions of surrounding tissues, thereby avoiding the problems of tissue damage and inflammatory pains caused by liposuction or low invasion fat-dissolving apparatus used in the prior art and the problems such as surrounding tissue inflammations and necrosis infections triggered by cell disruption and necrosis caused by components of a fat-dissolving injection, phosphatidylcholine or sodium deoxycholate.

## Description

### Field of the Invention

The present invention relates to a composition, and more particularly to a composition used for reducing localized fat and reducing weight. The present invention relates to an application of the composition, and more particularly to the application used for reducing localized fat and reducing weight. The present invention further relates to a pharmaceutical composition comprising the composition, and more particularly to the pharmaceutical composition used for reducing localized fat and reducing weight. The present invention further relates to an application, and more particularly to the application of the pharmaceutical composition used for reducing localized fat and reducing weight.

### Background of the Invention

With more and more people's change on the concept of beauty, and the increasing standards in their personal health, body shape, and weight, weight loss and body contouring are no longer issues cared only by obese individuals. Therefore, weight loss and fitness center and weight management market are booming, driving relative developments such as, diet foods, fitness products, weight loss clinical management, medical equipment, fitness equipment, etc. According to the statistic report "Global Weight Loss and Gain Market 2009-2014" issued by Markets and Markets, the global weight management market was estimated to be US $586.3 billion in 2014 and could grow into $650.9 billion in 2015; wherein the primary market is the United States, it's market scale was estimated to reach $310 billion, and the estimated market scale of Europe, the second largest market, is $238 billion. At the same time, due to the rise of consumer's concern of the awareness on health, especially on the after effects of chronic diseases caused by obesity, has made it an increasingly important agenda globally; therefore, besides targeting the obesity population, more and more people are actively developing on body weight control and body contouring for the healthy population, which has drawn strong attentions of all industries towards the body weight control and body contouring and led to the development boom in the industry, the above-mentioned reasons are all the main reasons why the market could grow steadily. The statistics of American Society of Plastic Surgeons (ASPS) issued in 2012 showed that the demand of body contouring has continuously ranked first in the field of plastic surgery and estimated demands would maintain an annual growth rate at about 12.3% until 2017. "Global Pipeline Analysis, Competitive Landscape and Market Forecasts" published by Research and Markets showed that global market of body contouring equipment reached $900 million in 2010 and estimated to increase into $2 billion in 2017. It is because the demand of the body contouring market is huge and keeps on growing every year, various ways of liposuction and lipolytic methods appear on the market. In contrast, many high-risk or unsafe medical treatments have also appeared one after another.
Conventional liposuction surgery was developed as early as in the 1970s; because of the way of liposuction by dry liposuction under negative pressure without any infusion solution, the subcutaneous nerves and blood vessels were damaged greatly; moreover, the amount of blood loss was large and the risk of surgery was high. Tumescent liposuction and super wet liposuction were developed later on. Tumescent liposuction is by adding anesthetic and vasoconstrictor into the infusion solution, even though, in this method, local anesthesia replaces intravenous anesthesia, large amount of infusion solution has caused the anesthetic requires at least 12 hours to be metabolized, and hence increased the possible risks of local anesthetic to human body. Recently, the method which doctors preferably choose to use on large amount of liposuction is super wet liposuction, its characteristic is that the volume exchange of infusion solution has to be equal to the extracted fat and the chance of overload infusion can be effectively reduced. However, clinically, there are limitations to the sites of administration, the sites are primarily the areas with large fat accumulation such as the abdomen, and the thighs etc; meanwhile, the efficacy and safety of liposuction depend entirely on the skill and the proficiency of the surgeon. The process of liposuction cause much harm to the body, time-consuming, and would still causes side effects such as, severe postoperative bruising, swelling, pain and sensory paralysis, scar tissue, unsmooth skin surface; the recovery period could range from four to six weeks. Due to the lengthy operation time of liposuction, the greater blood loss has directly increased its surgical risk. Therefore, various auxiliary liposuction instruments are being designed by every industry, and thus auxiliary methods of liposuction like ultrasound and laser were developed one after another, the key factor is still the skill of the performing surgeon. Furthermore, cases of tissue burn or poor efficacy occur frequently while using relative auxiliary liposuction instruments. In view of the drawback faced in liposuction, every industry was continuously making improvements in both technique and instrument; the aesthetic medical industry of the United States and Europe are focusing on the development of aesthetic medical equipment since the 1980s. In the field of body weight control and body contouring, the manufacturers have transformed their main appeal from traditional liposuction to minimally invasive and even non-invasive lipolysis instrument. The goals of lipolytic products or instruments are to improve the traditional faults of large blood loss, lengthy recovery period, postoperative scars, and try to be less invasive, high safety, convenient, smaller wound, and short recovery period as possible; at the same time, maintain the advantages of better efficacy and competitive price for it to enter the aesthetic medical market with the enough advantages.

There is a lipolytic method by using mesotherapy, which uses phosphatidylcholine or sodium deoxycholate as active ingredients to inject to the obese region to lyse fat. The structure of this kind of ingredients is similar to the ingredient of cell membrane; therefore, it could break down the cell membrane of adipocyte cells and trigger cell necrosis. Injecting this kind of medicine into the mesoderm would cause massive adipocyte cell necrosis and then cause fat release; due to the medicine does not possess specificity, namely, not only targeted on adipocytes, many normal surrounding cells would also be affected and thus cause necrosis, in addition, cell necrosis would cause the surrounding tissue to initiate a series of inflammation reaction, further causing administration site inflammation, severe pain, and swelling, and even causing the risks of local tissue necrosis or infection. Although lipolytic method by injection , comparing to using large liposuction instrument, could overcomes the limitation in administration site, one lipolysis injection treatment course may require several dozens of injections every 2 weeks and 1 to 6 treatment course(s) to reach the goal of lipolysis effect. Although anesthetic has been added to the injection solution, the injected site may still suffer from severe inflammation and pain after the anesthesia has subsided, in addition, the number of treatment and injection frequency also needs to be improved. Consider of the risks of severe postoperative pain, nerve paralysis, local tissue necrosis, or infections, the limited dosage in single injection, and restricted to be applied in face, most surgeons no longer inject the ingredients mentioned above to reduce localized fat of the patient; although the United States has approved the first lipolysis inject product made from sodium deoxycholate, it has a certain degree of side-effects and could only use in double chin, making its usage to be limited. Overall, a product that could effectively reduce localized fat, has lower side effects, safer is lacking on the current market; therefore, under the high demand of surgeons and consumers, it is a trend in the market to develop a localized lipolysis injection formulation that is safer, with low side effects, and could overcome the mentioned defects and limitations.

### SUMMARY OF THE INVENTION

The present invention provides a composition used for reducing localized fat and reducing weight, comprising a weight ratio of resveratrol to turmeric extract ranging from 1: 30 to 10: 1. Preferably, the weight ratio of resveratrol to turmeric extract is 1:19.

The composition of the present invention can inhibit adipocytes growth and induce planned adipocyte apoptosis to reach the effect of reducing localized fat deposits and decreasing adipocytes; it can also reduce body weight but does not cause the surrounding cells and the tissue inflammation or tissue necrosis, nor would it cause inflammation or severe pain to the surrounding tissues, thus avoids the tissue damage, inflammation, and pain caused by above mentioned liposuction surgery or low-invasive lipolysis instrument. Moreover, it would not cause necrosis nor result in problems such as surrounding tissue inflammation or infection in the surrounding tissues as triggered by necrosis caused by lipolysis injection composition ingredient phosphatidylcholine or sodium deoxycholate. The present invention can reduce weight as well.

According to the present invention, the term "turmeric extract" as used herein refers to the extract comprises curcumin, wherein the turmeric extract comprises 80% to 100% of curcumin.

The present invention also provides a preparation method of a composition comprising resveratrol and turmeric extract. The method comprises: mixing a composition with at least one of the pharmaceutically acceptable salts composition, pharmaceutically acceptable stabilizers or bacteriostatic agents or pharmaceutically acceptable emulsifiers, excipient such as surfactants, anesthetics, wherein the composition comprises resveratrol and turmeric extract; and
preparing the mixture into an injection formulation.

Preferably, the stabilizer comprises, but is not limited to, xylitol, sorbitol, polydextrose, isomalt or dextrose.

Preferably, the "pharmaceutically acceptable excipient" comprises, but is not limited to, lubricant, suspending agent, solubilizer, glidant, emulsifier or surfactant. The quantity of excipient required will depend upon the quantity of the active ingredient, and one excipient can perform one or more functions.

Preferably, the lubricant comprises but are not limited to, agar, calcium stearate, ethyl oleate, ethyl laurate, glycerin, glyceryl palmitostearate, hydrogenated vegetable oil, magnesium oxide, magnesium stearate, mannitol, poloxamer, ethylene glycol, sodium benzoate, sodium lauryl sulfate, sodium stearoyl acid, sorbitol, stearic acid, talc, or zinc stearate.

Preferably, the suspending agent comprises, but are not limited to, mannitol, carboxymethyl cellulose (CMC), CMC-Na.

Preferably, the solubilizer comprises, but are not limited to, hydroxypropyl-beta-cyclodextrin, tween 80, or castor oil.

Preferably, the glidant comprises, but are not limited to materials such as magnesium stearate, silicon dioxide, magnesium trisilicate, powdered cellulose, starch, talc, tribasic calcium phosphate, calcium silicate, magnesium silicate, Colloidal Silicon Dioxide, silicon hydrogel, etc.

Preferably, the emulsifier might be a naturally occurring phospholipid, comprising, but not limited to, soybean lecithin, lecithin, monoglycerides, diglycerides, sodium stearate, sorbitan esters of fatty acid, or polyoxyethylene sorbitan monooleate.

Preferably, the surfactant comprises, but are not limited to, Tween, polyethylene-polypropylene glycol, polyoxyethylene-monostearate, polyoxyethylene alkyl ethers (e.g. polyoxyethylene lauryl ether), Triton-X, polyoxyethylene-polyoxypropylene copolymer, or sodium dodecyl sulfate (SDS).

The present invention further provides an application of the composition in preparing a pharmaceutical composition which is used for reducing localized fat and reducing weight.

The present invention further provides a pharmaceutical composition used to reduce localized fat and reduce weight; wherein, the pharmaceutical composition comprising an effective dosage of the composition which is used for reducing localized fat and reducing weight and a pharmaceutically acceptable carrier.

The pharmaceutical composition of the present invention can exist in multiple forms, including, but not limited to, liquid, semi-solid and solid pharmaceutical forms. For example, liquid solutions (e.g. injectable and infusible solution), dispersions, suspensions, powders, lyophilized powders, or liposomes or transdermal ointment or patch. The preferred form depends on the expected mode of administration and therapeutic application. Preferably, the pharmaceutical composition of the present invention is in the form of infusible solutions, and the preferred administration mode is non-intestinal mode, such as injectable solution. In an embodiment of the present invention, the pharmaceutical composition comprises an effective dosage of resveratrol and turmeric extract composition for reducing localized fat is subcutaneously administered. Preferably, the pharmaceutical composition comprising an effective dosage of resveratrol and turmeric extract composition for reducing localized fat is administered to subcutaneous fat by subcutaneous injection. In an embodiment of the present invention, the formulation of the ointment is well known in the art, including, but not limited to, active agent, wax, water, petrolatum, preservatives, higher alcohols, polyhydric alcohols, emulsifiers, solvents, thickeners, plasticizers, fragrances, buffers, antibiotics, stabilizers or mixtures thereof.

The dosage of the pharmaceutical composition of the present invention can be adjusted accordingly to different parameters. The parameters include, but are not limited to the type of the subject, the weight of the subject, and the thickness and area of the localized fat of the subject. The pharmaceutical composition of the present invention can be administered once, multiple or continuously within 24 hours, and can also be administered multiple or continuously per week or per month. The method of administration is by injection, subcutaneous implantation, implantable infusion, ointment, or patch. Preferably, the method of injection includes, but is not limited to subcutaneous injection, subcutaneous implantation, intravenous drip, intravenous infusion pump method, implantable infusion pump method.

The present invention further provides an application of the pharmaceutical composition used for reducing localized fat and reducing weight, which comprises administering an effective dosage of the pharmaceutical composition comprising resveratrol and turmeric extract composition to a local sites of a subject, to make the local site of the subject achieve the effect of inhibit adipocyte growth, promote adipocyte apoptosis, decrease fat deposition, and reduce weight.

Preferably, the subjects are human or animals.

Preferably, the effective dosage of the pharmaceutical composition to be administered to the subject every time is from 0.4 mg/kg to 100 mg/kg. More preferably, the effective dosage of the pharmaceutical composition to be administered to the subject every time is from 1 mg/kg to 60 mg/kg.

Preferably, the term "reduce body weight" as used herein refers to the reduction of body weight gain of the subject by 5% to 30%.

According to the present invention, the term "reducing localized fat" as used herein refers to: after administering the effective dosage of compositions comprising resveratrol and turmeric extract of the present invention, adipocyte growth could be inhibited, adipocytes initiate procedural or planned apoptosis, and the localized deposit fat could be reduced. As shown in the embodiment of the present invention, the amount of localized fat reduction can be detected by administering a specific range of the dosage of the composition comprising resveratrol and turmeric extract, and measuring the growth inhibitory rate of adipocytes, cell apoptosis degree, and the change of the subcutaneous fat and visceral fat of rats in a specific period. Preferably, the localized fat includes, but is not limited to, subcutaneous fat, visceral fat, localized deposit fat, or adipocytes.

Preferably, the localized fat sites include, but are not limited to, face, jaw, arm, waist, abdomen or thighs.
The effect of the composition or the pharmaceutical composition of the present invention is significantly superior to that of resveratrol or turmeric extracts administered alone. Moreover, there has never been a precedent of resveratrol or turmeric extracts administered alone, or together, to reduce localized fat by localized injection. Furthermore, the present invention is primarily to inhibit adipocyte growth and promote adipocyte apoptosis to reduce localized fat by administrating medicine through localized injection, without affecting surrounding cells or tissues, and no significant side effect was found in animal assay. Therefore, the composition or the pharmaceutical composition of the present invention is safer with fewer side effects. The technical level of the composition or the pharmaceutical composition in the present invention is completely different from existing techniques or products. Thus, it could be used to avoid the problems of localized cells necrosis, severe pain, inflammation and necrosis and/or infection of surrounding tissues caused by conventional liposuction surgery, lipolysis instruments such as high-intensity focused ultrasound lipolysis instrument, or lipolytic injection ingredients such as phosphatidylcholine and sodium deoxycholate. Moreover, it can also significantly reduce postoperative bruising, swelling, pain and sensory paralysis, as well as shorten recovery period of the patient.

### BRIEF DESCRIPTION OF THE DRAWINGS

Fig. 1 is the bar chart presenting the effect of inhibiting preadipocytes growth in each group tested by MTT assay in the present invention.
Fig. 2 is the bar chart presenting the effect of inhibiting differentiating adipocytes growth in each group tested by MTT assay in the present invention;
Fig. 3 is the bar chart presenting the effect of the formulation UL003C and sodium deoxycholate on mature adipocytes growth inhibition tested by MTT assay in the present invention.
Fig. 4 is the bar chart presenting the effect of the formulations UL003A, UL003C and UL003R on adipocyte apoptosis promotion in the present invention obtained by labeling with Annexin V/PI and then detecting with flow cytometer.
Fig. 5 is the bar chart presenting the effect of the formulations UL003A, UL003C and UL003R on adipocyte apoptosis promotion in the present invention obtained by performing caspase 3 stain and then detecting with flow cytometer.
Fig. 6 is the bar chart presenting the effect of turmeric extract, resveratrol and the formulation UL003C on adipocyte apoptosis promotion in the present invention obtained by performing caspase 3 stain and then detecting with flow cytometer.
Fig. 7 is the bar chart presenting the effect of the formulation UL003C and sodium deoxycholate on adipocyte apoptosis promotion in the present invention obtained by labeling with Annexin V/PI and then detecting with flow cytometer.
Fig. 8 is the line graph presenting the weight change of rats in each group after the rats are fed with a high-fat diet to induce localized fat increasing and locally administered with medicine via injection in the present invention.
Fig. 9 is the bar chart presenting the subcutaneous fat of rats in each group after the rats are fed with a high-fat diet to induce localized fat increasing and locally administered with medicine via injection in the present invention.
Fig. 10A is the bar chart presenting the serum biochemical values (creatinine and glutamic oxaloacetic transaminase (GOT)) of rats in each group after the rats are fed with a high-fat diet to induce localized fat increasing and locally administered with medicine via injection in the present invention.
Fig. 10B is the bar chart presenting the serum biochemical values (urea and glutamic pyruvic transaminase (GTP)) of rats in each group after the rats are fed with high-fat diet to induce localized fat increasing and locally administered with medicine via injection in the present invention.
Fig. 11 is the bar chart presenting the expression of apoptosis related protein such as Bax and Bcl-2 and the ratio of Bax/Bcl-2 in each group detected by Western Blotting after the rats are fed with a high-fat diet to induce localized fat increasing and locally administered with medicine via injection in the present invention.

### DETAILED DESCRIPTION OF THE INVENTION

The technical means, accompanied by the drawings and the preferred embodiments of the present invention, taken to achieve the projected invention purpose could be further elaborated below.

### Embodiment 1: Preadipocyte growth inhibition assay

In this embodiment, 3T3-L1 preadipocytes were incubated in 96-well plates with 1x10⁴ cells per well. With the exception of the DMSO solution control group (control group), 50 ppm resveratrol, 50 ppm turmeric extract, 80 ppm green tea extract, and 100 ppm of the composition UL003A, UL003C, or UL003R in the present invention were added into different wells respectively. There were seven groups and three replicates were preformed in each group. After administration process and 48 hours incubation, photos were taken to record the cell's growth condition, and the growth inhibitory effect of 3T3-L1 preadipocytes in each group was analyzed by MTT assay. In composition UL003A, the weight ratio of resveratrol to green tea extract is 9: 1; in the composition UL003C, the weight ratio of resveratrol to turmeric extract is 1: 19; in the composition UL003R, the weight ratio of resveratrol to turmeric extract is 9: 1. Data of all groups are presented in Mean ± SD. The letters a, b, c, d, and e represent the results of the statistics. Different letters represent statistical difference among the groups (p<0.05).

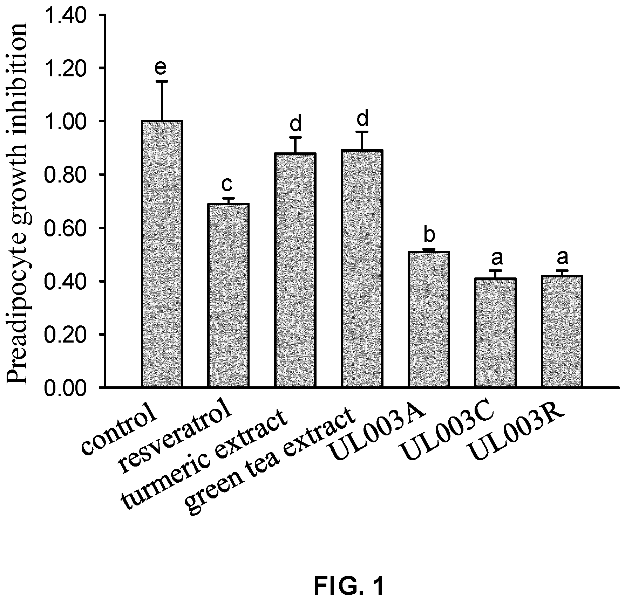

Results are shown as in Fig. 1, compared to the DMSO solvent control group, the compositions UL003A, UL003C, and UL003R of the present invention could all effectively inhibit preadipocytes growth (p<0.05); compared to the single plant extracts groups, the compositions UL003A, UL003C, and UL003R had better inhibitory effect, and the differences are significant (p<0.05).

### Embodiment 2: Differentiating adipocytes growth inhibition assay

In this embodiment, 3T3-L1 preadipocytes cells were incubated in 12-well plates with 1x10⁵ cells per well. The medium was replaced with medium containing 5 µg/ml of insulin differentiation agent, 1 µM of dexamethasone, 0.5 mM of 3-isobutyl-1-methylxanthine on the fourth day of incubation. DMSO, 50 ppm of resveratrol, 50 ppm of turmeric extract, 80 ppm of green tea extract, and 100 ppm of the composition UL003A, UL003C, or UL003R in the present invention were added to different wells respectively, wherein the group added with DMSO was control group. There were seven groups and three replicates were performed in each group. After administration process and 48 hours incubation, photos were taken to record the cell's growth condition, and the inhibitory effect of respective experimental matters on the differentiating adipocytes was analyzed by MTT assay. Data of all groups are presented in Mean ± SD. The letters a, b, c, d, e, and f represent the results of the statistics. Different letters represent statistical difference among groups (p<0.05).

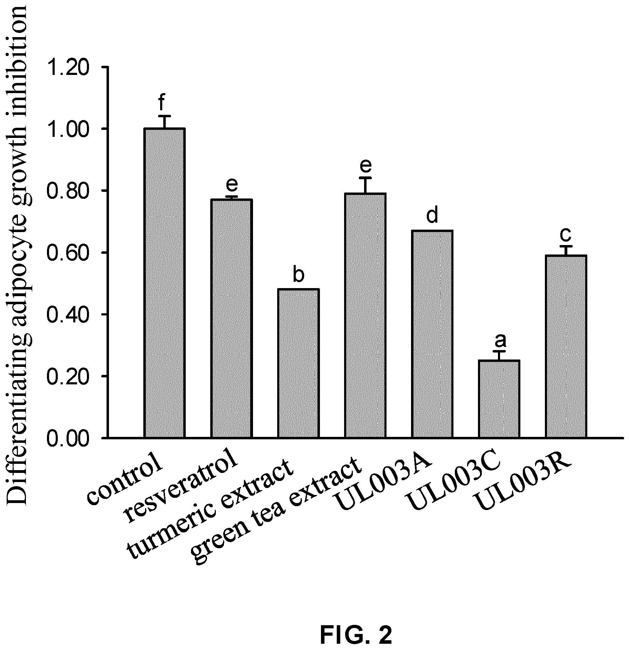

Results are shown as in Fig.2, compared to the DMSO solvent control group, all of the compositions UL003A, UL003C, and UL003R of the present invention could significantly inhibit differentiating adipocytes growth (p<0.05), wherein the composition UL003C had the best inhibitory effect on differentiating adipocytes growth. Compared to other single plant extract groups, the composition UL003C had better inhibitory effect on differentiating adipocyte growth.(p<0.05).

### Embodiment 3: Mature adipocytes growth inhibition assay

In this embodiment, the inhibitory effect of the composition of the present invention on mature localized adipocytes is compared with the inhibiting effect of the conventional sodium deoxycholate. In this embodiment, 3T3-L1 cells were incubated in 12-well plates with 3x10⁴ cells per well. The medium was replaced with an incubation fluid containing 5 µg/ml of insulin, 1 µM of dexamethasone, and 0.5 mM of 3-isobutyl-1-methylxanthine on the fourth day of incubation. After incubation for another two days, medium was renewed with an incubation fluid containing 5 µg/ml insulin to incubate for additional four days. When 3T3-L1 cell differentiation was completed, with the exception of the PBS solvent control group and the DMSO solvent control group, 50 ppm or 100 ppm of the composition UL003C or sodium deoxycholate were added to different wells respectively. There were six groups and three replicates were performed in each group. After administration process and 48 hours incubation, photos were taken to record the cell's growth condition, and the inhibitory effect of respective experimental matters on mature adipocytes was analyzed by MTT assay.

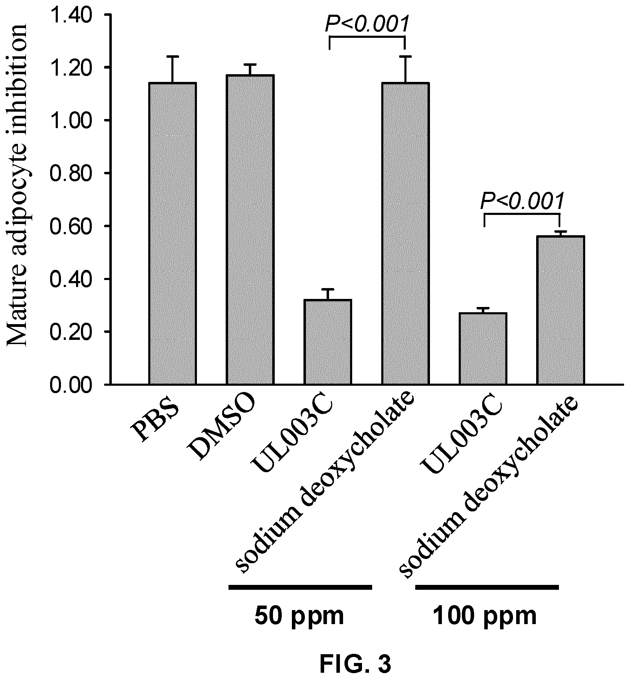

Results are shown as in Fig.3, the composition of the present invention could effectively inhibit mature adipocytes growth; at both of the two different dosages of 50 ppm and 100 ppm, the inhibitory effect of the composition UL0003C of the present invention on mature adipocytes was significantly greater than that of sodium deoxycholate (*p*<0.001).

### Embodiment 4: Adipocyte apoptosis assay (I)

In this embodiment, 3T3-L1 cells were incubated in 12-well plates with 1x10⁵ cells per well. The medium was replaced with an incubation fluid containing 5 µg/ml of insulin differentiation agent, 1 µM of dexamethasone, and 0.5 mM of 3-isobutyl-1-methylxanthine on the fourth day of incubation. After culturing the cells for four days in the incubation fluid containing differentiation agent and waiting until the adipocyte differentiation has been completed, with the exception of the DMSO solvent control group, 50 ppm of compositions UL003A, UL003C, and UL003R of the present invention were added to different wells respectively. There were four groups and three replicates were performed in each group. After administration process and 24 hours incubation, cells were collected and then stained with Annexin V/PI, and cell apoptosis was analyzed by flow cytometer; wherein Annexin V-PI-represents the number of survival mature adipocytes, and Annexin V+PI+ represents the number of apoptotic mature adipocytes; this method is used to distinguish and determine the apoptosis degree induced by the experimental matters in each group.

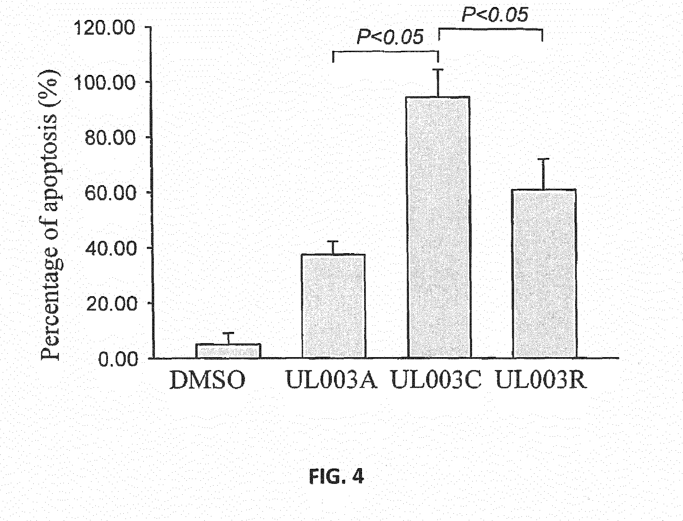

Results are shown as in Fig.4, after treating the mature adipocytes with the above mentioned experimental matters for 24 hours, all of the compositions UL003A, UL003C, and UL003R of the present invention could significantly induced mature adipocytes apoptosis (p<0.05) compared to the DMSO solvent control group, wherein composition UL003C has the best apoptotic effect on mature adipocytes which is significantly better than composition UL003A and UL003R (p<0.05).

### Embodiment 5: Adipocyte apoptosis assay (II)

In this embodiment, 3T3-L1 cells were incubated in 12-well plates with 1x10⁵ cells per well. The medium was replaced with an incubation fluid containing 5 µg/ml of insulin differentiation agent, 1 µM of dexamethasone, and 0.5 mM of 3-isobutyl-1-methylxanthine on the fourth day of incubation. After four days incubation in the incubation fluid containing differentiation agent and waiting until the adipocytes have matured, with the exception of the DMSO solvent control group, 50 ppm and 100 ppm of the compositions UL003A, UL003C, and UL003R of the present invention were respectively added to different wells. There were seven groups and three replicates were performed in each group. After administration process and 3 hours incubation, cells were collected and the caspase 3 stain was performed. Cell apoptosis was analyzed by flow cytometry, wherein if the caspase 3 of the cells have been labeled, it represents the cells initiated apoptosis; this method is used to compare the apoptosis degree induced by the experimental matters in each group under different dosage.

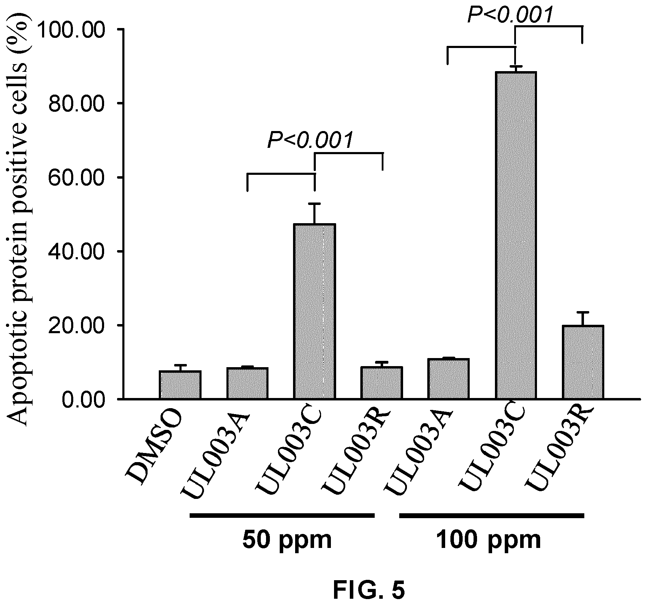

Results are shown as in Fig.5, compared to the DMSO solvent control group, no matter the mature adipocytes were treated with 50 ppm or 100 ppm of composition UL003C of the present invention, caspase 3-labeled cells in these groups were significantly more than that in the DMSO solvent control group (p<0.001). It indicates that the composition UL003C could significantly induce mature adipocytes apoptosis, and has the best effect.

### Embodiment 6: Adipocyte apoptosis assay (III)

In this embodiment, 3T3-L1 cells were incubated in 12-well plates with 1x10⁵ cells per well. The medium was replaced with an incubation fluid containing 5 µg/ml of insulin differentiation agent, 1 µM of dexamethasone, and 0.5 mM of 3-isobutyl-1-methylxanthine on the fourth day of incubation. After four days incubation in the incubation fluid containing differentiation agent and waiting until the adipocytes have matured, with the exception of the DMSO solvent control group, 50 ppm turmeric extract, 50 ppm resveratrol, and 50 ppm and 100 ppm of the composition UL003C in the present invention was added to each group respectively to perform the experiment; there were five groups and three replicates were performed in each group. According to previously published articles, cells treated with resveratrol for 16 hours were more likely to be labeled by caspase 3, cells in the remaining groups were treated with medicine and incubated for 3 hours. Cells were collected and perform caspase 3 stain, and cell apoptosis was analyzed by flow cytometry. Cells with labeled-caspase 3 represents the number of apoptotic cells; this method is used to compare the apoptosis degree induced by the experimental matters in each group under different dosage.

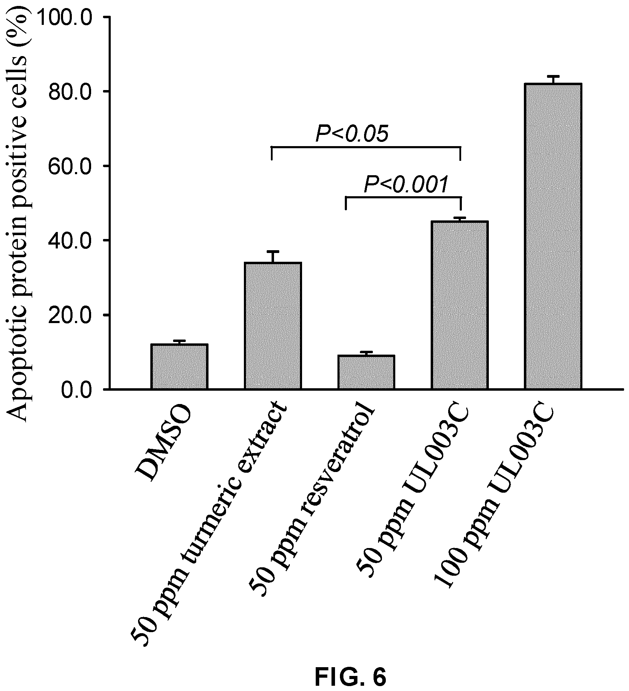

Results are shown as in Fig.6, after adipocytes were treated with 50 ppm of composition UL003C of the present invention, cells with labeled caspase 3 in these group were significantly more than that in the DMSO solvent control group, (p<0.001) and also significantly more than the turmeric extract group (p<0.05) and resveratrol group (p<0.001) with the same dosage. It indicates that the effect of the composition UL003C in the present invention on promoting mature adipocytes apoptosis is significantly superior to the effect of any single one plant extract ingredient in the composition.

### Embodiment 7: Adipocyte apoptosis assay (IV)

In this embodiment, the effect of composition UL003C in the present invention on mature adipocytes apoptosis promotion was compared with that of the well-known sodium deoxycholate. In this embodiment, 3T3-L1 cells were incubated in 12-well plates with 1x10⁵ cells per well. The medium was replaced with an incubation fluid containing 5 µg/ml of insulin differentiation agent, 1 µM of dexamethasone, and 0.5 mM of 3-isobutyl-1-methylxanthine on the fourth day of incubation. After four days incubation in the incubation fluid containing differentiation agent and waiting until the adipocyte cells matures, with the exception of the DMSO solvent control group, 100 ppm of composition UL003C and sodium deoxycholate were added to different wells respectively; there are three groups in the experiment, and three replicates were performed in each group. After administration process and 24 hours incubation, cells were collected and Annexin V/PI stain was performed. Cell apoptosis was analyzed by flow cytometry, wherein Annexin V-PI-cells represented the number of the survival mature adipocytes, and Annexin V+PI+ cells represented the number of the apoptotic mature adipocytes; this method is used to compare the apoptosis degree induced by the experimental matters in the two groups.

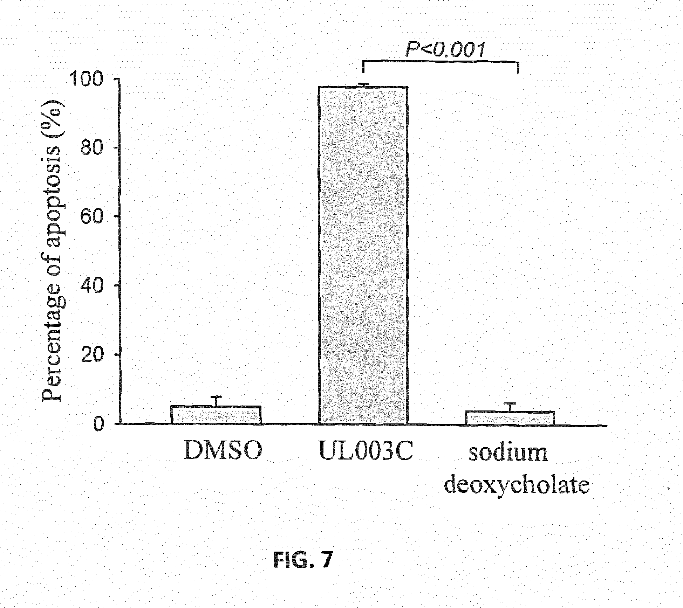

Results are, shown as in Fig.7, compared to the DMSO solvent control group and sodium deoxycholate with the same concentration, the composition UL003C could significantly induce mature adipocytes apoptosis (p<0.001). In contrast, there was no significant difference between the DMSO solvent control group and sodium deoxycholate group (p> 0.05). It indicates that composition UL003C in the present invention could significantly induce mature adipocytes apoptosis, and the known locally lipolytic ingredient sodium deoxycholate does not.

### Embodiment 8: Animal assay by using rats (I)

In this experiment, eight-week-old male Sprague-Dawley (SD) rats were used in this experiment. There are three groups, which are control group, UL003C-20 group (20 mg/kg BW, also known as low-dosage group), and UL003C-40 group (40 mg/kg BW, also known as high-dosage group) respectively. Four seven-week-old male rats were used in each group to perform the experiment, the initial weight of the rats were 207 g ± 6 g. Medication could be administered after the rats of all the groups were fed with high-fat diets consecutively for 2 weeks to make the subcutaneous fat of the rats thicken and make the rats weigh 330 g ± 10 g. Different dosage of the composition UL003C is subcutaneous injected to subcutaneous fat, the sites to be injected are the bilateral inguinal fat pads, two injection point on each side (5 mg/kg/point), and the total dosages of each rat were 20 mg/kg/time of the subject and 40 mg/kg/time of the subject respectively; the same volume, 4mL/kg/time, of water for injection was administered to the rats in control group. The injections were performed every other day for three times. Set the day on which the administrations were initiated as the first day, and repeatedly administered the medicine according to above mentioned method on the third day and the fifth day. The change in body weight and the average daily diet intake were recorded every day during the experiment period. After the rat's weight has been weighted for the last time on the twenty-first (21^{st}) day, the rats were fasted for 24 hours. Blood was taken to measure the liver and renal function index; the index includes glutamic pyruvic transaminase (GPT), glutamic oxaloacetic transaminase (GOT), creatinine and urea. After rats had been sacrificed, subcutaneous abdominal fat, fat in the superior inguinal region (superior groin), and fat in the inferior inguinal region (inferior groin) were obtained to weight the amount of the subcutaneous fat. Data of all groups are presented in Mean ± SD; letters refer to the statistic results, and different letters represent that there are statistical differences among groups (p<0.05), same letters represent that there is no statistical difference among groups (p>0.05).

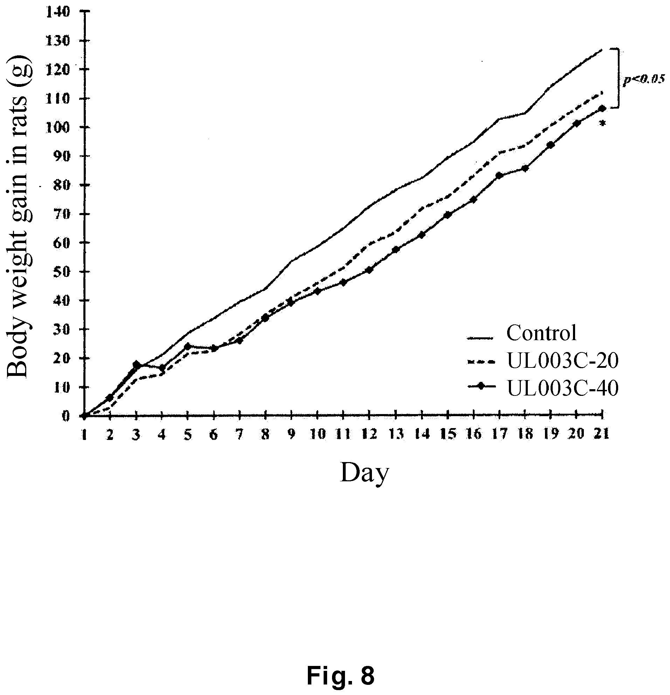

Results are shown as in Fig.8, the total weight gains of the rats treated with either low-dosage or high-dosage of the composition UL003C of the present invention were lower than the total weight gains of the rats in control group, wherein the amount of weight gain of rats in UL003C-40 group was significantly lower than the weight gain of the rats in control group (*p* <0.05), reduced by 15.8% of weight. Compare with the control group, although the amount of weight gain of rats in UL003C-20 group has a descending trend, decreased by 11.1%, but did not reach significant statistical difference (p>0.05). It indicates that the body weight can also be reduced by injecting the composition of the present invention to the subject, and the effect is related to the dosage.

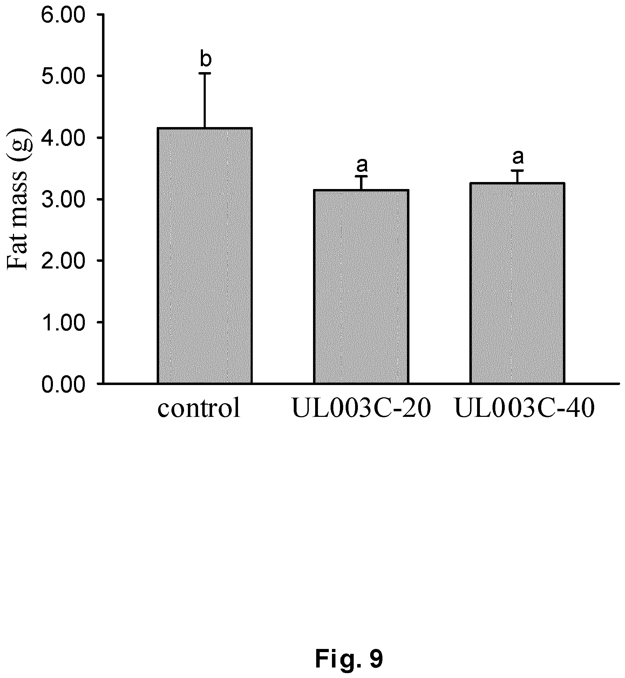

As shown in Fig.9, it is the result presenting the reduction of subcutaneous fat at the administration site of the rats in each group after sacrifice; compared to control group, the composition UL003C of the present invention could significantly reduce subcutaneous fat around the injection site(p<0.05); wherein in the low dosage group, the amount of subcutaneous fat reduction could reach 24.3% at the injection site (p<0.05) ; in the high dosage group, the amount of subcutaneous fat reduction reach 21.6% at the injection site (p<0.05).

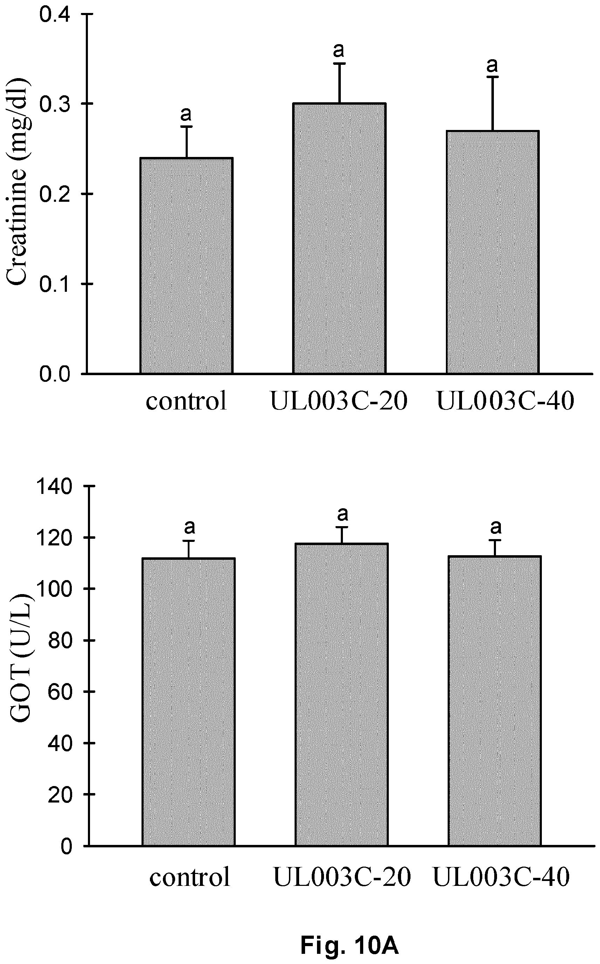

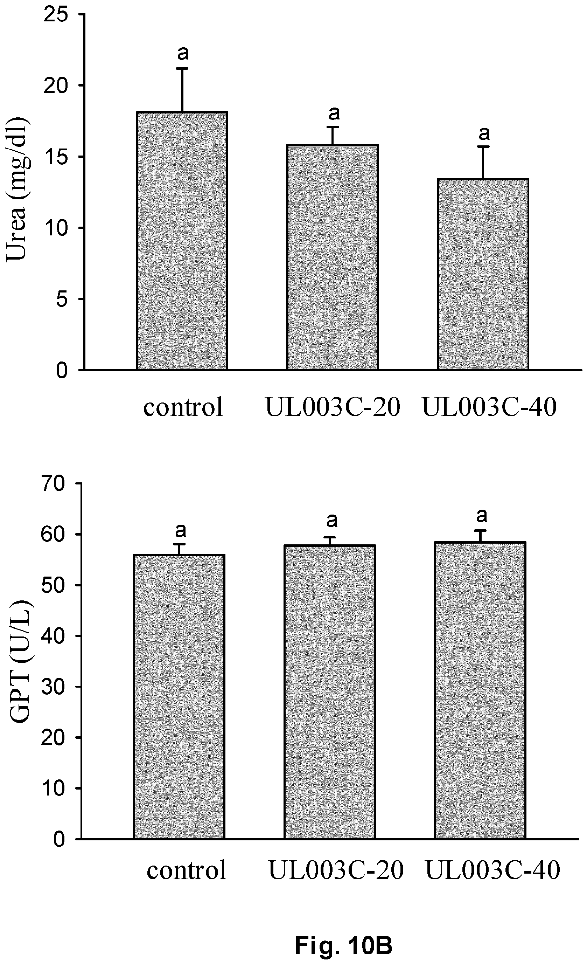

As shown in Figs. 10A and 10B, the serum biochemical values such as creatinine, urea, GOT, and GPT does not have statistical difference (*p>* 0.05). It indicates that the case of injecting either low-dosage or high-dosage of the composition UL003C of the present invention would not affect the safety index; that is, it indicates that the composition of the present invention has good security.

### Embodiment 9: Animal Assay by using rats (II)

Bcl-2 and Bax are two important regulators in apoptotic pathway, the balance between the two regulators is an important mechanism to regulate apoptosis. Higher Bcl-2 expression suppresses apoptosis while higher Bax expression promotes apoptosis. The high or low ratio between these two proteins could determine that the cells would tend to be survival or initiate apoptosis reaction.

In this experiment, the expression of apoptosis-inhibiting protein Bcl-2 and apoptosis-promoting protein Bax2 in adipose tissue of the subcutaneous injection site of rats in each group was determined by Western blot analysis, and the ratio of Bax and Bcl-2 was used to evaluate the effect of composition UL003C of the present invention on adipocyte apoptosis. In this experiment, the subcutaneous adipose tissues in the inferior inguinal region around the injection sites of the rats sacrificed in example 8 was used to perform protein extract with 450 µl T-PER®, 30 µg of protein of each group was taken to perform polyacrylamide gel electrophoresis (SDS-PAGE) and then transfer the protein to PVDF membrane. The Bcl-2 antibody, model number sc-7382, used in Western blot was purchased from Santa Cruz and, the Bax antibody, model number sc- 526, was purchased from Santa Cruz. Data of all groups are presented in Mean ± SD; letters refer to the statistic results, and different letters represents that there is statistical difference among groups (p<0.05), same letters represents that there is no statistical difference among groups (p>0.05).

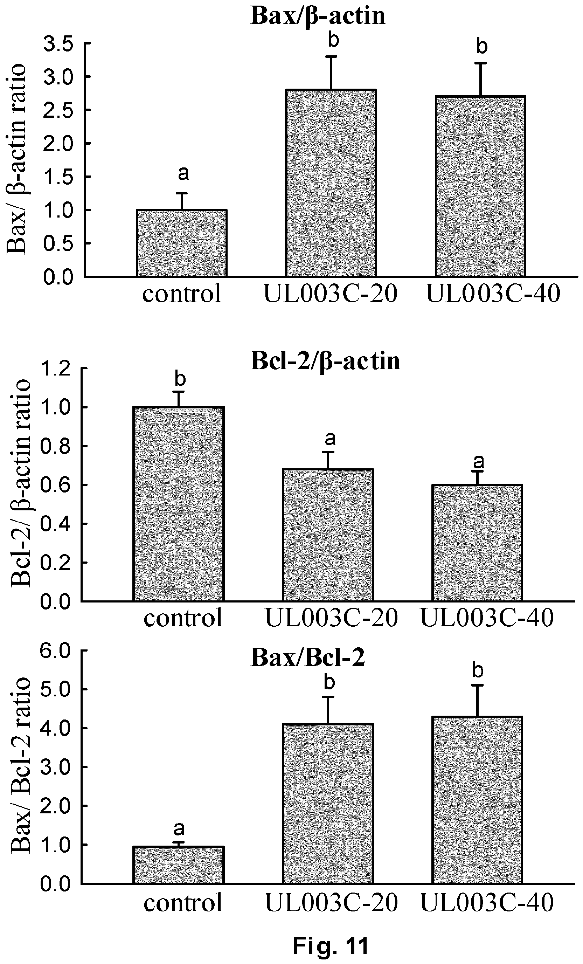

Results are shown as in Fig. 11, compared with control group, either low-dosage or high-dosage of the composition UL003C of the present invention could significantly enhance the Bax expression (p<0.05) and significantly inhibit Bcl-2 expression (p<0.05), and its Bax/Bcl-2 ratio was significantly higher than that of control group (p<0.05). The results indicate that injecting the composition UL003C of the present invention to the rats could effectively induce the adipocytes in the adipose tissue initiating apoptosis. The present invention could indeed and effectively induce adipocyte to initiate apoptosis and reduce its amount of localized fat. The results of animal assays reconfirmed the mechanism that the composition of the present invention could achieve the result of reducing adipocytes and localized fat is via the pathway of promoting adipocyte apoptosis.
The above descriptions are merely the preferred embodiments, and are not limitation to the present invention in any form; even though the preferred embodiments of the present invention are disclosed above, it is not used to limit the present invention by any means. Any technical personnel, as long as it is within the range, without deviation, of the technical skill plan of the present invention, could use the above disclosed technical information to make an embodiment of equal effect with several adjustments or modification, but any information that has not deviated from the technical plans of the present invention, which are any simple modification from the techniques of the present invention made substantially to the above embodiments, still belongs within the range of the technical plan s of the present invention.

### Examples of the present disclosure may be described by the following numbered paragraphs:

1. A composition used for reducing topical fat and reducing weight; characterized in that, the composition comprises a weight ratio of resveratrol to turmeric extract ranging from 1: 30 to 10: 1.
2. The composition as set out in paragraph 1; characterized in that, the weight ratio of resveratrol to turmeric extract is 1: 19.
3. An application of the composition as set out in paragraph 1 or 2 in preparing a pharmaceutical composition used for reducing topical fat and reducing body weight.
4. A pharmaceutical composition used for reducing topical fat and reducing body weight; characterized in that, the pharmaceutical composition comprises an effective amount of the composition as set out in paragraph 1 or 2 and a pharmaceutically acceptable carrier.
5. An application of the pharmaceutical composition as set out in paragraph 4 in preparing a pharmaceutical composition for reducing topical fat and reducing weight; characterized in that, administering an effective amount of the pharmaceutical composition to a topical site of a subject to make the topical site of the subject achieve the effect of reducing topical fat and reducing weight.
6. The application as set out in paragraph 5; characterized in that, the subject is animal or human.
7. The application as set out in paragraph 5; characterized in that, the topical site comprises face, jaw, arm, waist, abdomen or thighs, etc.
8. The application as set out in paragraph 5; characterized in that, the administering method is by injection, subcutaneous implantation, implantable infusion, ointment or patch.
9. The application as set out in paragraph 8; characterized in that, the injection is subcutaneous injection.
10. The application as set out in paragraph 9; characterized in that, the subcutaneous injection is injected to subcutaneous fat layer.
11. The application as set out in paragraph 8; characterized in that, the effective amount of the pharmaceutical composition is to administrate a dosage of 0.4 mg/kg to 100 mg/kg pharmaceutical composition to the subject each time.
12. The application as set out in paragraph 11; characterized in that, the effective amount of the pharmaceutical composition is to administer a dosage of 1 mg/kg to 60 mg/kg pharmaceutical composition to the subject each time.

## Claims

1. A composition comprising a weight ratio of resveratrol to turmeric extract ranging from 1:30 to 10:1 wherein the turmeric extract comprises 80% to 100% curcumin for use in a therapeutic method of reducing localized fat in a subject, wherein the method comprises administering the composition to a localized fat site of the subject such that the localized fat is reduced.

2. A composition comprising a weight ratio of resveratrol to turmeric extract ranging from 1:30 to 10:1 wherein the turmeric extract comprises 80% to 100% curcumin for use in a therapeutic method of reducing body weight of a subject, wherein the method comprises administering the composition to a localized fat site of the subject such that body weight of the subject is reduced.

3. A composition comprising a weight ratio of resveratrol to turmeric extract ranging from 1:30 to 10:1 wherein the turmeric extract comprises 80% to 100% curcumin for use in a method of treating obesity of a subject, wherein the method comprises administering the composition to a localized fat site of the subject such that the localized fat is reduced.

4. A composition comprising a weight ratio of resveratrol to turmeric extract ranging from 1:30 to 10:1 wherein the turmeric extract comprises 80% to 100% curcumin for use in a method of treating obesity of a subject, wherein the method comprises administering the composition to a localized fat site of the subject such that body weight of the subject is reduced.

5. A composition comprising a weight ratio of resveratrol to turmeric extract ranging from 1: 30 to 10: 1 for use as a lipolytic medicament in a method of reducing localized fat in a subject, wherein the turmeric extract comprises 80% to 100% curcumin, and wherein the method comprises administering the lipolytic medicament to a localized fat site of the subject such that localized fat at the localized fat site is reduced.

6. A composition comprising a weight ratio of resveratrol to turmeric extract ranging from 1: 30 to 10: 1 for use as a lipolytic medicament in a method of reducing body weight of a subject, wherein the turmeric extract comprises 80% to 100% curcumin, and wherein the method comprises administering the lipolytic medicament to a localized fat site of the subject such that the body weight of the subject is reduced.

7. The composition for the use according to any of the preceding claims, wherein the subject is a human or animal subject.

8. The composition for the use according to any of the preceding claims, wherein the localized fat site is selected from face, jaw, arm, waist, abdomen or thigh.

9. The composition for the use according to any of the preceding claims, wherein the method comprises administration by injection, subcutaneous implantation, implantable infusion, ointment or patch.

10. The composition for the use according to claim 9, wherein the injection is subcutaneous injection, and optionally wherein the subcutaneous injection is injected to subcutaneous fat layer.

11. The composition for the use according to any of the preceding claims, wherein the method comprises administering a dosage of 0.4 mg/kg to 100 mg/kg, or optionally a dosage of 1 mg/kg to 60 mg/kg.

12. A composition comprising a weight ratio of resveratrol to turmeric extract ranging from 1: 30 to 10:1, wherein the turmeric extract comprises 80% to 100% curcumin, optionally wherein the weight ratio of resveratrol to turmeric extract ranges from 1:30 to 1:19.

13. The composition for the use according to any of claims 1 to 11, or the composition according to claim 12, wherein the composition further comprises a pharmaceutically acceptable carrier or excipient.

14. The composition for the use according to any one of claims 1 to 11, or the composition according to any one of claims 12-13, wherein the weight ratio of resveratrol to turmeric extract ranges from 1: 30 to 9:1.

15. The composition for the use according to any one of claims 1 to 11, or the composition according to any one of claims 12-13, wherein the weight ratio of resveratrol to turmeric extract is 1: 19.
